Europäisches Patentamt

European Patent Office

Office européen des brevets

(11) Numéro de publication: **0 272 970 B1**

(19)

(12) **FASCICULE DE BREVET EUROPEEN**

(45) Date de publication de fascicule du brevet:
30.10.91

(51) Int. Cl.⁵: **B01J 27/053**, C07C 2/16

(21) Numéro de dépôt: 87402753.5

(22) Date de dépôt: 03.12.87

(54) Procédé de préparation d'un catalyseur renfermant un composé du nickel déposé sur alumine et utilisation du catalyseur obtenu pour la dimérisation des oléfines.

(30) Priorité: 17.12.86 FR 8617785

(43) Date de publication de la demande:
29.06.88 Bulletin 88/26

(45) Mention de la délivrance du brevet:
30.10.91 Bulletin 91/44

(84) Etats contractants désignés:
BE DE GB IT NL

(56) Documents cités:
EP-A- 0 174 836
GB-A- 1 215 943
US-A- 3 959 400

(73) Titulaire: **INSTITUT FRANCAIS DU PETROLE**
**4, Avenue de Bois-Préau**
**F-92502 Rueil-Malmaison(FR)**

(72) Inventeur: **Chauvin, Yves**
**64, avenue du Général Leclerc**
**F-78230 le Pecq(FR)**
Inventeur: **Commereuc, Dominique**
**32, rue Abel Vacher**
**F-92190 Meudon(FR)**
Inventeur: **Hugues, François**
**10, Rue du Clos Chaland**
**F-69390 Charly(FR)**
Inventeur: **Saussine, Lucien**
**2, Place des Frères Tissandier**
**F-78290 Croissy sur Seiner(FR)**

## Description

L'objet de la présente invention est un procédé de préparation d'un catalyseur de dimérisation des oléfines, de sélectivité améliorée, et de l'utilisation du catalyseur obtenu.

La dimérisation et l'oligomérisation des oléfines légères à l'aide de catalyseurs consistant en divers composés du nickel déposés sur supports minéraux est bien connue. En particulier le brevet US 2,794,842 décrit la polymérisation de l'éthylène en présence de sulfate de nickel déposé sur silice-alumine. Le brevet US 3,959,400 décrit un procédé de dimérisation du propylène à l'aide d'un catalyseur composé de sulfate de nickel déposé sur alumine. Le nickel et le soufre sont introduits en proportions équimoléculaires (S/$_{Ni}$ = 1) et le rapport S/Ni est toujours supérieur à 0,95 dans le catalyseur final.

Si de tels catalyseurs ont une activité élevée, ils présentent néanmoins l'inconvénient d'être peu sélectifs en dimères, en particulier à conversion élevée où le taux de trimères et de tétramères peut représenter plus de 30 % des produits.

Ceci présente un inconvénient quand ce sont les dimères qui sont recherchés, en particulier dans le cas de la dimérisation du propylène pour l'obtention d'essence ; l'indice d'octane de cette essence étant d'autant plus médiocre que la teneur en trimères et tétramères est plus élevée.

Selon la présente invention on obtient une composition catalytique améliorée en opérant de la façon suivante :

   a) on met en contact de l'alumine avec une source d'ions sulfate et une source d'ions nickel en solution aqueuse ou organique, les proportions d'ions sulfate et d'ions nickel étant choisies telles que l'on introduit les-dits ions dans l'alumine en proportions correspondant à un rapport atomique S : Ni compris entre 0,4 : 1 et 0,8 : 1,

   b) on élimine le solvant de l'alumine qui a absorbé les-dits ions dans les proportions ci-desssus et,

   c) on la chauffe entre 300 et 800° C en atmosphère non réductrice.

Dans le catalyseur obtenu, le rapport atomique S/$_{Ni}$ est lui même de préférence compris entre 0,4 : 1 et 0,8 : 1, le soufre et le nickel pouvant être dosés par spectroscopie de fluorescence X.

L'étape finale de la préparation étant un chauffage en atmosphère non réductrice, on admet que dans le catalyseur, le soufre se présente entièrement sous forme sulfate ; dans ce qui suit, on mentionnera donc parfois le rapport molaire

$$\frac{sulfate,}{nickel}$$

considéré comme égal au rapport atomique $\frac{S}{Ni}$.

## Description détaillée

L'oléfine employée peut être l'éthylène, le propylène ou l'un des isomères des butènes. Cette oléfine peut être mise en oeuvre à l'état pur ou en mélange avec un ou plusieurs composés ne réagissant pas sur le catalyseur dans les conditions utilisées, tels que les hydrocarbures saturés cycliques ou acycliques, en particulier ceux ayant de 2 à 10 atomes de carbone. La concentration de l'oléfine dans le mélange peut être de 5 à 100 %, de préférence de 10 à 50 % en poids. On préfère utiliser une charge sensiblement exempte d'eau, ce qui peut être réalisé par exemple en séchant la charge au préalable sur tamis moléculaire 3 A : une teneur en eau inférieure à 100ppm, et plus particulièrement inférieure à 10ppm est préférée.

La réaction de dimérisation est de préférence effectuée en continu, le catalyseur étant disposé en lit fixe dans un tube et étant traversé par le mélange réactionnel. La pression est maintenue de telle façon que le mélange soit à l'état liquide ; cette pression dépend donc de la température de réaction, de l'oléfine employée et de sa concentration dans le mélange. La vitesse volumique horaire est habituellement choisie entre 0,1 et 10h$^{-1}$, selon les conversions désirées, pour un volume de catalyseur égal à l'unité. La température est utilement choisie entre 10° C et 150° C ; il est préférable de la maintenir entre 30 et 60° C.

Le catalyseur peut être préparé selon toute technique connue, adaptée toutefois à l'obtention d'un catalyseur ayant un rapport atomique S : Ni compris entre 0,4 : 1 et 0,8 : 1. Trois modes opératoires préférés sont indiqués ci-après :

   1) imprégnation de l'alumine, préalablement séchée, avec une solution contenant à la fois un sel de nickel décomposable par la chaleur en oxyde de nickel et du sulfate de nickel dans des proportions telles que l'on ait un rapport atomique S : Ni compris entre 0,4 : 1 et 0,8 : 1.

   2) imprégnation de l'alumine avec une solution contenant un sel de nickel décomposable par la chaleur en oxyde de nickel, suivie d'un séchage et d'une calcination du solide puis d'une imprégnation du solide calciné avec une solution contenant des ions sulfate. Les quantités respectives de sel de nickel et d'ions sulfate sont choisies de telle manière que l'on ait un rapport atomique S/Ni inférieur à 0,8 et supérieur à 0,4.

   3) préparation d'oxyde de nickel supporté sur alumine par le processus décrit en 2), puis imprégnation du solide calciné avec une solution diluée d'acide sulfurique de manière que le rapport atomique $\frac{S}{Ni}$ soit inférieur à 0,8 et supérieur à 0,4.

Lors des imprégnations telles que décrites cidessus, on peut opérer "à sec" c'est à dire avec un volume de liquide au maximum égal au volume poreux du support, ou avec un excès de solution. Après les phases d'imprégnation telles que décrites ci-dessus, le catalyseur est séché et calciné. Le séchage peut être effectué par exemple par passage d'un courant d'air sec, la teneur en eau étant de préférence inférieure à 100ppm, à travers le solide à 100-150°C pendant 0,5 à 6 heures. La calcination peut être réalisée par chauffage en atmosphère non réductrice, à des températures comprises entre 300°C et 800°C, de préférence entre 400 et 500°C, pour une durée comprise par exemple entre 0,5 et 12 heures.Après calcination, le catalyseur est utilisable pour effectuer la réaction de dimérisation.

Le sel de nickel décomposable en oxyde par la chaleur, à une température au plus égale à 600°C, est, par exemple, le nitrate de nickel ou un composé organique, par exemple le formiate, l'oxalate, l'acétylacétonate ou le 2-éthyl-hexanoate de nickel.

Le solvant selon le sel choisi, peut être par exemple l'eau, un alcool ou un hydrocarbure.

L'alumine peut être par exemple une alumine gamma ou êta. L'alumine présente avantageusement une surface spécifique d'au moins 20m²g⁻¹ et un volume poreux d'au moins 0,1 cm³g⁻¹. On peut par exemple l'utiliser sous forme de billes ou d'extrudés.

La teneur en nickel du catalyseur est comprise par exemple entre 0,5 et 15 % en poids, et de préférence entre 2 et 8 %. Le rapport atomique $\frac{S}{Ni}$ est pris entre 0,4 : 1 et 0,8 : 1.

Les exemples suivants illustrent l'invention.

EXEMPLE N°1

40 g d'alumine (de surface spécifique 238 m²g⁻¹ et de volume poreux 0,52 cm³g⁻¹), sous forme de billes de diamètre 0,2-0,4mm, préalablement calcinée sous air sec à 500°C pendant 3 heures est ensuite imprégnée à sec, à 25°C, avec 20 ml d'une solution contenant 4,9 g de Ni(NO₃)₂, 6H₂O et 3,8g de NiSO₄, 6H₂O. Après séchage sous air à 150°C pendant 2 heures, le solide est calciné sous air sec à 500°C pendant 3 heures. L'analyse du solide par fluorescence X à ce stade indique qu'il contient 4,07 % en poids de nickel et 1,23 % de soufre soit un rapport atomique S/Ni égal à 0,46. Le solide est placé dans un tube en acier inoxydable servant de réacteur. Une solution, maintenue sous une pression de 4 bars et contenant 10 % en poids de propylène dans le n-heptane, est envoyée à travers le lit de catalyseur maintenu à 40°C, avec une vitesse volumique horaire de 0,82h⁻¹. A la sortie du réacteur, l'effluent est analysé par chromatographie en phase vapeur ; l'analyse indique que la conversion du propylène est de 73 % et la sélectivité en hexènes est de 94,4 %.

EXEMPLE N°2

40 g d'alumine , de même nature que dans l'exemple n°1 et traitée de la même manière, est imprégnée à sec avec une solution contenant 9,0g de Ni(NO₃)₂, 6H₂O. Le solide obtenu est séché sous air à 150°C pendant 2 heures puis calciné sous air sec à 500°C pendant 3 heures. Le solide calciné est ensuite imprégné à sec avec une solution contenant 1,7g de (NH₄)₂SO₄. Le solide est séché sous air à 150°C puis calciné sous air sec à 500°C pendant 3 heures. L'analyse montre qu'il contient 4,0 % Ni et 1,1 % S, soit S/Ni = 0,41. Le catalyseur obtenu est soumis à un test de dimérisation du propylène dans les mêmes conditions que dans l'exemple n°1 avec cependant une vitesse volumique horaire de 0,77h⁻¹. La conversion du propylène est de 64,6 % et la sélectivité en hexènes de 94,8 %.

EXEMPLE N°3

40 g d'alumine est imprégnée à sec avec une solution contenant 8,5 g de Ni(NO₃)₂, 6H₂O et le solide séché et calciné comme dans l'exemple n°2. Le solide est ensuite imprégné à sec avec une solution contenant 0,015 mole d'H₂SO₄, puis séché et calciné comme dans l'exemple n°2. L'analyse montre qu'il contient 3,82 % Ni et 1,04 % S, soit $\frac{S}{Ni}$ = 0,50. Le test de dimérisation du propylène, effectué dans les mêmes conditions que dans l'exemple n°1, avec une vitesse volumique horaire de 0,81h⁻¹, donne une conversion du propylène de 68 % et une sélectivité en hexènes de 92,5 %.

EXEMPLE N°4

40 g d'alumine est imprégnée à sec comme dans l'exemple n°1 avec une solution contenant 3,1g de Ni(NO₃)₂ 6H₂O et 4,7 g de Ni SO₄, 6H₂O. Après séchage et calcination effectués comme dans l'exemple n°1, le solide, contenant 3,72 % Ni et 1,52 % S soit $\frac{S}{Ni}$ = 0,63, conduit, dans le test de dimérisation du propylène, effectué comme dans l'exemple n°1 avec une vitesse volumique horaire de 0,78h⁻¹, à une conversion de 82,3 % et à une sélectivité en hexènes de 85 %.

EXEMPLE N°5

40 g d'alumine est imprégnée à sec comme dans l'exemple n° 1 avec une solution contenant 2,2 g de Ni(NO$_3$)$_2$, 6H$_2$O et 5,5 g de NiSO$_4$, 6H$_2$O. Après séchage et calcination le solide, contenant 3,73 % Ni et 1,80% S, soit $\frac{S}{Ni}$ = 0,74, conduit, dans le test de dimérisation du propylène effectué avec une vitesse volumique horaire de 0,76h$^{-1}$, à une conversion de 94,4 % et à une sélectivité en hexènes de 79 %.

## EXEMPLE N°6 (comparatif)

12 g d'alumine est imprégnée à sec avec une solution aqueuse contenant 2,2 g de NiSO$_4$, 6H$_2$O. Le solide est séché, calciné, analysé, il contient alors 3,82 % Ni et 2,08 % S soit S/Ni = 1,0 puis testé dans la dimérisation, la vitesse volumique horaire étant de 2,61h$^{-1}$. La conversion obtenue est de 64,8 % et la sélectivité en hexènes de 74 %.

## EXEMPLE N°7 (comparatif)

40 g d'alumine est imprégnée à sec avec une solution aqueuse contenant 7,4 g de NiSO$_4$, 6H$_2$O. Le solide est séché, calciné, analysé. Il contient alors 3,84 % Ni et 2,10 % S soit $\frac{S}{Ni}$ = 1,0 puis testé dans la dimérisation du propylène, la vitesse volumique horaire étant de 0,79h$^{-1}$. La conversion est de 97,0 % et la sélectivité en hexènes de 66 %.

Les exemples 1 à 4 comparés aux exemples 6 à 7 montrent que les catalyseurs tels que le rapport atomique S/Ni est inférieur à 0,8 et supérieur à 0,4 sont plus sélectifs en hexènes que ceux où le rapport $\frac{S}{Ni}$ est égal à l'unité.

## Revendications

1. Procédé de préparation d'un catalyseur renfermant de l'alumine, et du nickel dont la teneur est comprise entre 0,5 et 15 % en poids, comprenant les étapes suivantes :

   a) on met en contact de l'alumine avec une source d'ions sulfate et une source d'ions nickel en solution,
   b) on sèche l'alumine qui a absorbé lesdits ions dans les proportions ci-dessus et :
   c) on la chauffe entre 300 et 800°C en atmosphère non réductrice.

   caractérisé en ce que, au cours de l'étape a) les proportions d'ions sulfate et d'ions nickel sont choisies telles que l'on introduit lesdits ions dans l'alumine en proportions atomiques S:Ni comprises entre 0,4:1 et 0,8:1.

2. Procédé selon la revendication 1, dans lequel la source d'ions nickel et d'ions sulfate comprend du sulfate de nickel et un sel décomposable par la chaleur.

3. Procédé selon la revendication 2, dans lequel le sel de nickel décomposable par la chaleur est le nitrate de nickel.

4. Procédé selon l'une des revendications 1 à 3, dans lequel on utilise une solution contenant à la fois les ions sulfate et les ions nickel.

5. Procédé selon l'une des revendications 1 à 3, dans lequel on met l'alumine d'abord en contact avec une solution aqueuse contenant un sel de nickel décomposable par la chaleur, on sèche et on calcine le solide obtenu, puis on met ce solide en contact avec une solution aqueuse contenant des ions sulfate.

6. Utilisation du catalyseur obtenu par le procédé de l'une des revendications 1 à 5 dans la dimérisation d'un hydrocarbure mono-oléfinique.

## Claims

1. Process for the preparation of a catalyst containing alumina and nickel whereof the amount is comprised between 0.5 and 15 % by weight, which process comprises :

   a) contacting the alumina with compounds providing sulphate and nickel ions in solution,
   b) drying the alumina which has absorbed the said ions in the proportions mentioned above,
   c) heating the alumina between 300 and 800°C in a non-reducing atsmosphere,

   characterized in that, in step (a) the proportions of sulphate and nickel ions are chosen in order to introduce the said ions into the alumina in an atomic proportion S : Ni from 0.4:1 to 0.8:1.

2. The process of claim 1 wherein the nickel and the sulphate ion source contains nickel sulphate and a nickel salt which can be decomposed by heat.

3. The process of claim 2 wherein the nickel salt that can be decomposed by the heat is nickel nitrate.

4. The process of one of claims 1 to 3 wherein a solution containing both sulphate ions and nickel ions is used.

5. The process of one of claims 1 to 3 wherein the alumina is first contacted with an aqueous solution containing a nickel salt that can be decomposed by heat, then the obtained solid is dried and calcinated before it is contacted with an aqueous solution containing sulphate ions.

6. Use of the catalyst prepared following the process of one of claims 1 to 7 for the dimerization of a mono-olefinic hydrocarbon.

**Patentansprüche**

1. Verfahren zum Herstellen eines Katalysators, der enthält: Aluminiumoxid und Nickel, dessen Gehalt zwischen 0,5 und 15 Gew.-% liegt, die folgenden Stufen umfassend:
   a) man kontaktiert Aluminiumoxid mit einer Sulfationenquelle und einer Nickelionenquelle in Lösung,
   b) man trocknet das Aluminiumoxid, welches diese Ionen absorbiert hat, in den unten angegebenen Anteilen, und:
   c) man erwärmt zwischen 300 und 800° C in nicht reduzierender Atmosphäre,
   dadurch gekennzeichnet, daß während der Stufe a) die Anteile an Sulfationen und Nickelionen derart gewählt werden, daß man diese Ionen in das Aluminiumoxid in Atomanteilen S:Ni zwischen 0,4:1 und 0,8:1 einführt.

2. Verfahren nach Anspruch 1, bei dem die Nickelionen- und Sulfationenquelle Nickelsulfat sowie ein in der Wärme zersetzbares Salz umfaßt.

3. Verfahren nach Anspruch 2, bei dem das durch die Wärme zersetzbare Nickelsalz Nikkelnitrat ist.

4. Verfahren nach einem der Ansprüche 1 bis 3, bei dem man eine gleichzeitig die Sulfationen und die Nickelionen enthaltende Lösung verwendet.

5. Verfahren nach einem der Ansprüche 1 bis 3, bei dem man das Aluminiumoxid zunächst in Kontakt mit einer wässrigen Lösung bringt, die ein durch die Wärme zersetzbares Nickelsalz enthält, bei dem man trocknet und den erhaltenen Feststoff kalziniert und dann diesen Feststoff in Kontakt mit einer wässrigen Sulfationen enthaltenden Lösung setzt.

6. Verwendung des nach dem Verfahren eines der Ansprüche 1 bis 5 erhaltenen Katalysators zur Dimerisierung eines monoolefinischen Kohlenwasserstoffs.